# FASCICULE DE BREVET EUROPEEN

(11) **EP 1 793 834 B1**
(45) Date de publication et mention de la délivrance du brevet: **29.10.2008**
(21) Numéro de dépôt: 05805779.5
(22) Date de dépôt: 19.09.2005
(51) Int. Cl.: A61K 31/70, A61K 31/40, C07H 5/04

(54) **DERIVES PYRIDINIQUES D ' INDOLIN-2-ONE , LEUR PREPARATION ET LEUR APPLICATION EN THERAPEUTIQUE**
INDOLIN-2-ONE-PYRIDIN-DERIVATE, HERSTELLUNG UND THERAPEUTISCHE VERWENDUNG DAFÜR
INDOLIN-2-ONE PYRIDINE DERIVATIVES, PREPARATION AND THERAPEUTIC USE THEREOF

(30) Priorité: 20.09.2004 FR 0409906
(43) Date de publication de la demande: 13.06.2007
(73) Titulaire: Sanofi-Aventis, 75013 Paris (FR)
(72) Inventeur: FOULON, Loïc, F-31120 Portet sur Garonne (FR); SERRADEIL-LE GAL, Claudine, F-31750 Escalquens (FR); VALETTE, Gérard, F-31120 Lacroix-Falgarde (FR)
(74) Mandataire: Kugel, Dominique
(86) Numéro de dépôt international: PCT/FR2005/002316
(87) Numéro de publication internationale: WO 2006/032771

(56) Documents cités:
- EP-A- 0 212 481
- EP-A- 0 636 608
- WO-A-01/74775
- WO-A-95/18105

## Description

La présente invention a pour objet des dérivés pyridiniques d'indolin-2-one, leur préparation et leur application en thérapeutique.

Plusieurs composés ayant une activité sur les récepteurs de l'ocytocine sont connus. Des dérivés d'indolin-2-one, ligands des récepteurs de l'ocytocine, ont notamment été décrits dans les documents WO 01/74776, EP 636 608 or WO 95/18105.

D'autres dérivés d'indolin-2-one sont connus dans des indications différentes ; ils sont par exemple décrits comme antagonistes du calcium dans EP 212 481.

Toutefois, il existe toujours une nécessité de trouver de nouveaux ligands affins et sélectifs des récepteurs de l'ocytocine. Il a maintenant été trouvé des dérivés pyridiniques d'indolin-2-one, ligands affins et sélectifs des récepteurs de l'ocytocine.

La présente invention a pour objet des composés répondant à la formule (1) : 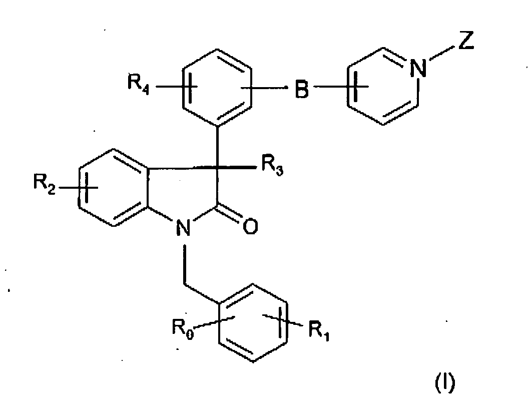 dans laquelle :
- R₀ représente un atome d'hydrogène, un atome d'halogène, un groupe (C₁-C₆)alkyle, un groupe (C₁-C₆)alcoxy ;
- R₁ représente un atome d'hydrogène, un atome d'halogène, un groupe (C₁-C₆)alkyle, un groupe (C₁-C₆)alcoxy ;
- R₂ représente un atome d'hydrogène, un atome d'halogène, un groupe (C₁-C₆)alkyle, un groupe (C₁-C₆)alcoxy ;
- R₃ représente un atome d'hydrogène, un groupe (C₁-C₆)alkyle ;
   - un atome d'oxygène,
   - un groupe (C₁-C₆)alkyle éventuellement substitué par un ou plusieurs groupes hydroxyle, (C₁-C₆)alcoxy , -COOR dans lequel R représente un atome d'hydrogène ou un groupe (C₁-C₃)alkyle, -CONRR' dans lequel R et R' représentent indépendamment l'un de l'autre un atome d'hydrogène ou un groupe (C₁-C₃)alkyle ou bien R et R' forment ensemble avec l'atome d'azote auquel ils sont liés un groupe pyrrolidinyle, pipéridinyle ou morpholinyle ;
   - un groupe tétrahydrofuranyle ou un groupe tétrahydropyranyle, lesdits groupes étant éventuellement substitués par un ou plusieurs groupes (C₁-C₃)alkyle, hydroxyle, carboxyle ou un groupe -CH₂OH ;
- B représente un groupe T-W, dans lequel :
   T représente un groupe -(CH₂)ₘ-, m pouvant être égal à 0 ou 1 ;
   W représente :
      - un groupe -CONR₆R₇ dans lequel :
         - R₆ représente un atome d'hydrogène, un groupe (C₁-C₆)alkyle,
         - R₇ représente un groupe (C₁-C₆)alkylène ;
      - un groupe -NR₈COR₉ dans lequel :
         - R₈ représente un atome d'hydrogène, un groupe (C₁-C₆)alkyle,
         - R₉ représente un groupe -(CH₂)ₙ-, n pouvant prendre des valeurs de 0 à 3 ; à l'état de base ou de sel pharmaceutiquement acceptable, à l'état d'hydrate ou de solvate, ainsi que ses énantiomères, diastéréoisomères et leurs mélanges.

Les composés de formule (1) peuvent comporter un ou plusieurs atomes de carbone asymétriques. Ils peuvent donc exister sous forme d'énantiomères ou de diasteréoisomères. Ces énantiomères, diastéréoisomères, ainsi que leurs mélanges, y compris les mélanges racémiques font partie de l'invention.

Les composés de l'invention peuvent également exister à l'état de bases ou de sels d'addition à des acides, à l'état d'hydrate ou de solvate.

Dans le cadre de la présente invention, on entend par :
- un atome d'halogène : un fluor, un chlore, un brome ou un iode,
- un groupe (C₁-C₃)alkyle et un groupe (C₁-C₆)alkyle : un groupe aliphatique saturé linéaire ou ramifié, comportant respectivement de 1 à 3 ou de 1 à 6 atomes de carbone. On peut citer par exemple, les groupes méthyle, éthyle, propyle, iso-propyle, butyle, iso-butyle, tert-butyle, pentyle, lesdits groupes alkyles précités pouvant être éventuellement substitués,
- un groupe (C₁-C₆)alkylène : un groupe alkyle divalent saturé, linéaire ou ramifié comportant de 1 à 6 atomes de carbone. On peut citer par exemple, les groupes méthylène, éthylène, 1-méthyléthylène, propylène,
- un groupe (C₁-C₆)alcoxy : un groupe aliphatique saturé linéaire ou ramifié pouvant comprendre de 1 à 6 atomes de carbone tel que défini précédemment, lié à un atome d'oxygène. On peut citer par exemple un groupe méthoxy ou éthoxy,
- un groupe tétrahydrofuranyle : un groupe tétrahydrofuran-2-yle ou tétrahydrofuran-3-yle, lesdits groupes précités pouvant être éventuellement substitués,
- un groupe tétrahydropyranyle : un groupe tétrahydropyran-2-yle, tétrahydropyran-3-yle, tétrahydropyran-4-yle, lesdits groupes précités pouvant être éventuellement substitués.

Parmi les composés objet de l'invention, on peut citer un premier groupe de composés de formule (1) dans laquelle :
- Ro représente un groupe (C₁-C₆)alcoxy, notamment un groupe méthoxy en position 2 du phényle ;
- R₁ représente un groupe (C₁-C₆)alcoxy, notamment un groupe méthoxy en position 4 du phényle ;
- R₂, R₃, R₄, Z et B sont tels que définis précédemment ;
à l'état de base ou de sel pharmaceutiquement acceptable, à l'état d'hydrate ou de solvate, ainsi ses énantiomères, diastéréoisomères et leurs mélanges.

Parmi les composés objet de l'invention, on peut citer un second groupe de composés de formule (I) dans laquelle :
- R₀ représente un atome d'hydrogène, un atome d'halogène, un groupe (C₁-C₆)alkyle, un groupe (C₁-C₆)alcoxy ;
- R₁ représente un atome d'hydrogène, un atome d'halogène, un groupe (C₁-C₆)alkyle, un groupe (C₁-C₆)alcoxy ;
- R₂, R₃, R₄ et B sont tels que définis précédemment ;
- Z représente un atome d'oxygène ;
à l'état de base ou de sel pharmaceutiquement acceptable, à l'état d'hydrate ou de solvate, ainsi ses énantiomères, diastéréoisomères et leurs mélanges.

Parmi les composés objet de l'invention, on peut citer un troisième groupe de composés de formule (I), dans laquelle :
- R₀ représente un atome d'hydrogène, un atome d'halogène, un groupe (C₁-C₆)alkyle, un groupe (C₁-C₆)alcoxy ;
- R₁ représente un atome d'hydrogène, un atome d'halogène, un groupe (C₁-C₆)alkyle, un groupe (C₁-C₆)alcoxy ;
- R₂, R₃, R₄ et B sont tels que définis précédemment ;
- Z représente :
   - un groupe (C₁-C₆)alkyle éventuellement substitué par un ou plusieurs groupes hydroxyle, (C₁-C₆)alcoxy , -COOR dans lequel R représente un atome d'hydrogène ou un groupe (C₁-C₃)alkyle, -CONRR' dans lequel R et R' représentent indépendamment l'un de l'autre un atome d'hydrogène ou un groupe (C₁-C₃)alkyle ou bien R et R' forment ensemble avec l'atome d'azote auquel ils sont liés un groupe pyrrolidinyle, pipéridinyle ou morpholinyle ;
   - un groupe tétrahydrofuranyle notamment un groupe tétrahydrofuran-2-yle, ou un groupe tétrahydropyranyle notamment un groupe tétrahydropyran-2-yle, lesdits groupes étant éventuellement substitués par un ou plusieurs groupes (C₁-C₃)alkyle, hydroxyle, carboxyle ou un groupe -CH₂OH ;
à l'état de base ou de sel pharmaceutiquement acceptable, à l'état d'hydrate ou de solvate, ainsi que ses énantiomères, diastéréoisomères et leurs mélanges.

Parmi les composés objet de l'invention, on peut citer un quatrième groupe de composés choisis parmi :
- Chlorure de 3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-1-(2-hydroxy-éthyl)-pyridinium,
- Chlorure de 1-carboxyméthyl-3-[({4-chloro-3-[5-chloro-1-(2,4-diméthoxy-benzyl)-3-méthyl-2-oxo-2,3-dihydro-1*H-*indol-3-yl]-benzoyl}-éthyl-amino)-méthyl]-pyridinium,
- Chlorure de 1-(β-D-glucopyranuronosyl))-3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-pyridinium,
- Chlorure de 3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-1-(D-glucopyranosyl)-pyridinium,
- Chlorure de 3-[({4-chloro-3-[5-chloro-1-(2,4-diméthoxy-benzyl)-3-méthyl-2-oxo-2,3-dihydro-1*H-*indol-3-yl]-benzoyl}-éthyl-amino)-méthyl]-1-(D-galactopyranosyl)-pyridinium,
- 4-Chloro-3-[5-chloro-1-(2,4-diméthoxy-benzyl)-3-méthyl-2-oxo-2,3-dihydro-1H-indol-3-yl]-N-éthyl-N-(1-oxy-pyrid-3-ylméthyl)-benzamide,
- Chlorhydrate du 4-chloro-3-[5-chloro-1-(2,4-diméthoxy-benzyl)-3-méthyl-2-oxo-2,3-dihydro-1H-indol-3-yl]-N-éthyl-N-(1-oxy-pyrid-3-ylméthyl)-benzamide,
- (+)-4-Chloro-3-[5-chloro-1-(2,4-diméthoxy-benzyl)-3-méthyl-2-oxo-2,3-dihydro-1H-indol-3-yl]-N-éthyl-N-[3-(1-oxy-pyridin-4-yl)-propyl]-benzamide.

L'invention a également pour objet un procédé de préparation des composés de formule (I).

Selon un mode de préparation général des composés de formule (I), on fait réagir le composé de formule (II) suivante : 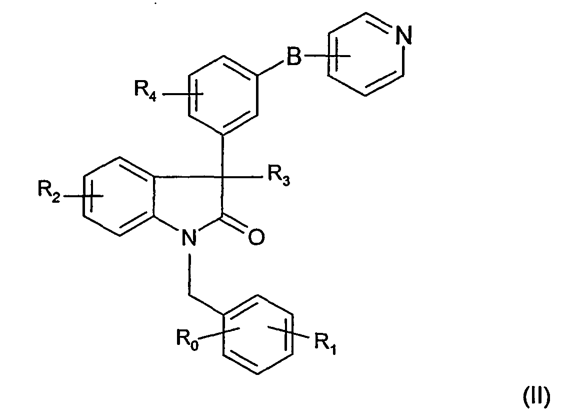 dans laquelle R₀, R_{1,} R_{2,} R_{3,} R₄ et B sont tels que définis précédemment, avec un peracide ou un composé de formule Z-X, dans lequel Z est tel que défini précédemment et X représente un groupe partant.

On entend par groupe partant, un groupe pouvant être remplacé facilement par un autre groupe lors d'une réaction de substitution par exemple. Des exemples de groupes partants ainsi que des références de préparation sont données dans « Advances Organic Chemistry », J. March, 3rd Edition, Wiley Interscience, pp. 310-316. A titre indicatif, on peut citer un atome d'halogène ou un groupe hydroxyle activé tel qu'un mésylate, tosylate, triflate, acétate, un sulfonate ou un imidate.

Les composés précurseurs de formule (II) sont décrits dans le document WO 01/74775. Lorsque les composés de formule (II) ne sont pas décrit spécifiquement dans le document WO 01/74775, ces derniers sont préparés par analogie avec les méthodes décrites dans le document WO 01/74775 ou selon des méthodes décrites dans la littérature et connues de l'homme du métier.

Ainsi, pour obtenir un composé de formule (I) dans laquelle Z représente un atome d'oxygène, on fait réagir un composé de formule (II) telle que définie précédemment, avec un dérivé oxydant tel qu'un peracide organique aliphatique ou un peracide aromatique. Parmi les peracides organiques aliphatiques on peut citer par exemple les acides peracétique, performique, perlaurique ou perphtalique. Parmi les peracides aromatiques, on peut citer par exemple les acides perbenzoïques ou leurs dérivés tel que l'acide 3-chloroperbenzoïque. La réaction se déroule à des températures comprises entre 0°C et 90°C, de préférence à des températures inférieures à 50°C.

Ces acides peuvent être utilisés tels quels lorsqu'ils sont stables ou générés *in situ* par réaction du peroxyde d'hydrogène sur l'acide ou l'anhydride carboxylique considéré. Ainsi, l'acide peracétique est obtenu par réaction d'une solution aqueuse de peroxyde d'hydrogène à 30-40% sur l'acide acétique glacial, l'acide perphtalique ou l'acide permaléique, par réaction du peroxyde d'hydrogène sur les anhydrides correspondants. On peut également procéder selon les méthodes décrites dans « Heterocyclic N-oxydes », CRC Press, (1991), Albini A., Pietra S.

Les peracides sont notamment choisis parmi des peracides anhydres et utilisés dans des solvants anhydres tels que les hydrocarbures aliphatiques, aromatiques, les solvants halogénés, le dioxane ou le diméthoxyéthane, les nitriles aliphatiques ou aromatiques.

Les composés finaux sont isolés tels quels ou salifiés par réaction avec un acide fort, tels que l'acide chlorhydrique, l'acide bromhydrique, l'acide sulfurique ou un acide sulfonique tel que l'acide méthane sulfonique par exemple, dans des solvants tels que les éthers, les alcools ou leur mélange.

Pour obtenir un composé de formule (I) dans laquelle Z représente :
- un groupe (C₁-C₆)alkyle éventuellement substitué par un ou plusieurs groupes hydroxyle, (C₁-C₆)alcoxy , -COOR dans lequel R représente un atome d'hydrogène ou un groupe (C₁-C₃)alkyle, -CONRR' dans lequel R et R' représentent indépendamment l'un de l'autre un atome d'hydrogène ou un groupe (C₁-C₃)alkyle ou bien R et R' forment ensemble avec l'atome d'azote auquel ils sont liés un groupe pyrrolidinyle, pipéridinyle ou morpholinyle, on fait réagir un composé de formule (II) avec un dérivé réactif de Z : Z-X dans lequel Z est tel que défini dans ce qui précède et X présente un groupe partant tel qu'un atome d'halogène, un triflate ou un sulfonate. La réaction se déroule à des températures comprises entre 0°C et 110°C, dans des solvants tels que les hydrocarbures aromatiques, les éthers aliphatiques, les alcools, de préférence dans les cétones tel que l'acétone, les amides aprotiques tel que la diméthylformamide et les nitriles aliphatiques tel que l'acétonitrile.

Les fonctions susceptibles de réagir telles que les amines, les alcools ou les acides carboxyliques, sont protégées par un groupe protecteur, puis déprotégées après réaction selon les méthodes bien connues de l'homme de l'art.

Par groupe protecteur, on entend un groupe qui permet d'une part de protéger une fonction réactive telle qu'un groupe hydroxyle, carboxyle ou une amine pendant une synthèse et d'autre part de régénérer la fonction réactive intacte en fin de synthèse. Des exemples de groupes protecteurs ainsi que les méthodes de protection et déprotection sont données dans Protective groups in Organics Synthesis, T.W. Greene et P.G.M. WUTS, 3nd Ed. (John Wiley & Sons, Inc., New York, 1999).

Les contre-ions générés par la réaction peuvent être conservés ou échangés en opérant par exemple sur des résines échangeuses d'ions.

On peut également procéder selon les méthodes décrites dans « The Chemistry of Heterocyclic Compounds, Pyridine and its derivatives », Part two (Quaternary Pyridinium Compounds) Interscience Publishers (1962).

Pour obtenir un composé de formule (I) dans laquelle Z représente :
- un groupe tétrahydrofuranyle ou un groupe tétrahydropyranyle, lesdits groupes étant éventuellement substitués par un ou plusieurs groupes (C₁-C₃)alkyle, hydroxyle, carboxyle, ou un groupe -CH₂OH
on opére selon le principe décrit précédemment décrit, notamment en faisant réagir un composé de formule (II) telle que définie précédemment, avec un dérivé réactif de Z : Z-X dans lequel Z est tel que défini ci-dessus et X représente un groupement partant tel qu'un halogène, un groupe triflate, acétate, imidate, thioalkyle ou thioaryle selon les méthodes décrites par G.S. Boons, Tetrahedron, (1996), 52, pp. 1095-1121.
La réaction se déroule à des températures comprises entre 50 et 110°C dans des solvants rigoureusement anhydres tel que l'acétonitrile (JOC, (2000), 65, pp. 8197-8203) ou dans du nitrométhane en présence de carbonate de cadmium tel que décrit dans J. Med. Chem., (1988), 31, pp. 1295-1305 et dans Chem. Pharm. Bull., (1987), 35(9), pp. 3975-3978.

Dans le cas où Z présente des fonctions susceptibles de réagir telles que les fonctions hydroxyles, ces dernières sont protégées par des groupes acyles tels que l'acétyle ou des groupes benzoyles, selon les techniques classiques, connues de l'homme du métier. Les fonctions hydroxyle sont ensuite déprotégées, soit en milieu acide avec une solution d'acide bromhydrique dans un alcool (JOC, (2000), 65, pp. 8197-8203) ou d'acide tétrafluroborique dans du méthanol *(*J.A.C.S., (1988), 120(16), pp. 3887-3893) ou trifluorométhane sulfonique dans de l'acétonitrile (Bloorg. Med. Chem. Lett., (2002), 12(8), pp. 1135-1138) soit en milieu basique avec une solution d'ammoniaque dans du méthanol *(*Chem. Commun., (1999), 8, pp. 729-730 et J. Med. Chem., (1988), 31 pp. 1295-1305).

On peut également opérer par saponification classique dans un alcool contenant de la soude aqueuse suivie d'une acidification avec de l'acide chlorhydrique.

Les contre-ions générés par la réaction peuvent être conservés ou échangés en opérant par exemple sur des résines échangeuses d'ions.

Les exemples suivants illustrent la présente invention, sans en limiter la portée. Dans ce qui suit :
- PF = Point de Fusion (en degré Celsius) mesuré sur banc Kofler.
- SM = Spectre de Masse réalisé sur un spectromètre quadripolaire de type Ptatform LCZ (WATERS) ou de type ZQ 4000 (WATERS) en mode d'ionisation par électrospray positif.
- RMN = résonnance magnétique nucléaire dont le spectre est réalisé sur un spectromètre à transformé de Fourier (BRUKER), à la température de 300K.
- s = singulet,
- t = triplet,
- m = multiplet,
- DMSO = diméthylsulfoxyde,
- CDCl₃ = chloroforme deutéré

### Exemple 1 :

Chlorure de 3-[({4-chloro-3-[5-chloro-1-(2,4-diméthoxy-benzyl)-3-méthyl-2-oxo-2,3-dihydro-1*H-*indol-3-yl]-benzoyl}-éthyl-amino)-méthyl]-1-(2-hydroxy-éthyl)-pyridinium
(I): R₀= 2-MeO ; R₁ = 4-MeO ; R₂= 5-Cl ; R₃= Me ; R₄= 4-Cl ;

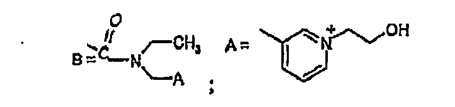

### Etape 1.1:

4-Chloro-3-[5-chloro-1-(2,4-diméthoxy-benzyl)-3-méthyl-2-oxo-2,3-dihydro-1*H*-indol-3-yl]-*N*-éthyl-*N*-pyridin-3-ylméthyl-benzamide

On traite l'acide 4-chloro-3-[5-chloro-1-(2,4-diméthoxybenzyl)-3-méthyl-2-oxoindolin-3-yl]benzoïque sous forme racémique, par la N-éthyl-3-pyridylméthylamine (document WO 01/074775, exemple 201 a) et b)). L'acide 4-chloro-3-[5-chloro-1-(2,4-diméthoxybenzyl)-3-méthyl-2-oxoindolin-3-yl] benzoïque sous forme racémique, est décrit dans le document WO 01/074775, à l'exemple 101. On obtient après chromatographie sur silice en éluant par un mélange dichorométhane - méthanol 90/10 le composé attendu sous forme racémique, cristallisé dans du méthanol (poudre blanche).

PF = 98°C (0,5 H₂O)

### Etape 1.2 :

Chlorure de 3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-1-(2-hydroxy-éthyl)-pyridinium

On chauffe à reflux pendant 3 jours sous atmosphère inerte le mélange de 0,2 g de l'amide préparé à l'étape 1.1 en présence de 0,54 g de 2-chloroéthanol dans 2 ml d'acétonitrile. Après évaporation du solvant sous vide, on purifie par chromatagraphie préparative sur silice en phase inverse C18 en éluant avec un gradient acétonitrile-eau de 20/80 à 80/20 et lyophilise la solution obtenue après congélation.
On obtient le produit attendu sous forme de poudre blanche.

SM [(+)ESI, m/z] = 648 (M⁺)
RMN ¹H 250MHz (CDCl₃) : 1,35 (t, 3H); 1,81 (s,3H) ; 3,61 (m, 2H) ; 3,62 (s, 3H) ; 3,90 (s, 3H) ; 4,10 (t, 2H) ; 4,70-5,2 (m, 6H) ; 6,4-9 (m, 13H).

### Exemple 2 :

Chlorure de 1-carboxyméthyl-3-[({4-chloro-3-[5-chloro-9-(2,4-diméthoxy-benzyl)-3-méthyl-2-oxo-2,3-dihydro-1*H*-indol-3-yl]-benzoyl}-éthyl-amino)-méthyl]-pyridinium
(I): R₀ = 2-MeO ; R₁ = 4-MeO ; R₂ = 5-Cl ; R₃ = Me ; R₄ = 4-Cl ;

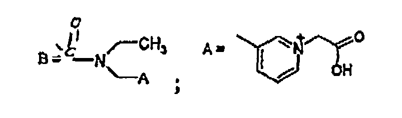

On obtient le composé attendu dans les mêmes conditions que celles de l'exemple 1 à partir du 4-chloro-3-[5-chloro-1-(2,4-diméthoxy-benzyl)-3-méthyl-2-oxo-2,3-dihydro-1H-indol-3-yl]-N-éthyl-N-pyridin-3-ylméthyl-benzamide sous forme racémique, préparé à l'étape 1.1 de l'exemple 1 et d'acide chloroacétique.

SM [(+)ESI, m/z] = 662 (M⁺).
RMN¹H 400MHz (CDCl₃) _{'} 1,27 (t, 3H) ; 1,80 (s, 3H) ; 3,52 (m, 2H) ; 3,80 (s, 3H) ; 3,88 (s, 3H) ; 4,76 (d, 1H) ; 4,90 (s, 2H) ; 5,12 (d, 1H) ; 5,47 (s, 2H) ; 6,40-9,4 (m, 13H).

### Exemple 3 :

Chlorure de 1-(β-D-glucopyranuronosyl))-3-[({4-chloro-3-[5-chloro-1-(2,4-diméthoxy-benzyl)-3-méthyl-2-oxo-2,3-dihydro-1*H*-indol-3-yl-benzoyl}-éthyl-amino)-méthyl]-pyridinium
(I): R₀ = 2-MeO ; R₁ = 4-MeO ; R₂ = 5-Cl ; R₃ = Me ; R₄ = 4-Cl ;

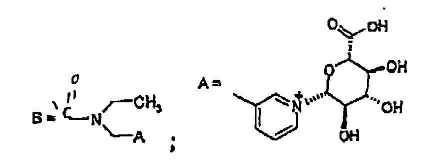

### Etape 3.1 :

Bromure de 3-[({4-Chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-1(méthyl-2,3,4-triacétyl-β-D-glucopyranuronosyl)pyridinium

On chauffe à reflux pendant 10 minutes sous atmosphère inerte, 100 mg du composé de l'exemple 201 décrit dans le brevet WO 01/074775 en présence de 131 mg d'acétobromo-α-D-glucuronate de méthyle et de 43 mg de carbonate de cadmium dans 1 ml de nitrométhane. Après évaporation du solvant sous vide, on chromatographie sur silice en éluant avec un mélange dichloromethane-méthanol 95/5. On obtient le produit attendu sous forme de poudre orangée.

SM [(+)ESI, m/z] = 920 (M⁺).

### Etape 3.2 :

Chlorure de 1-(β-D-glucopyranuronosyl))-3-[({4-chloro-3-[5-chloro-1-(2,4-diméthoxy-benzyl)-3-méthyl-2-oxo-2,3-dihydro-1*H*-indol-3-yl]-benzoyl}éthyl-amino)-méthyl]-pyridinium

On agite à 20°C pendant 15 minutes la solution de 100 mg du composé obtenu à l'étape 3.1, dans 1,6 ml de méthanol en présence de 1 ml de soude (2M). On ajoute 3,3 ml d'acide chlorhydrique (1N ; pH=4) à 10 °C. On évapore à sec puis purifie par chromatographie préparative sur silice en phase inverse C18 en éluant avec un gradient acétonitrile-eau de 40/60 à 80/20 puis lyophilise la solution obtenue après congélation. On obtient le produit attendu sous forme de poudre blanche.
SM [(+)ESI, m/z] = 780 (M⁺).
RMN¹H 400MHz (DMSO-d6) : 1,17 (t, 3H) ; 1,75 (s, 3H) ; 3,3-3,6 (m, 5H) ; 3,75 (s, 3H); 3,83 (s, 3H) ; 4,65-5,5 (m, 5H) ; 5,77 (d, 1H, J=8.51Hz) ; 6,48-9,2 (m, 13H).

### Exemple 4 :

Chlorure de 3-[({4-chloro-3-[5-chloro-1-(2,4-diméthoxy-benzyl)-3-méthyl-2-oxo-2,3-dihydro-1*H*-indol-3-yl]-benzoyl}-éthyl)-amino)-méthyl]-1-(D-glucopyranosyl)-pyridinium
(I): R₀ = 2-MeO ; R₁ = 4-MeO ; R₂ = 5-Cl ; R₃ = Me ; R₄ = 4-Cl ;

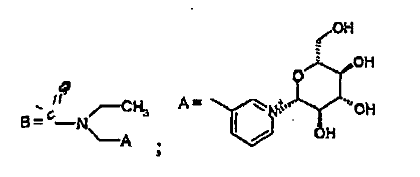

On obtient le produit attendu dans des conditions opératoires analogues à celles décrites à l'exemple 3 de la présente invention, à partir du 4-chloro-3-[5-chloro-1-(2,4-diméthoxy-benzyl)-3-méthyl-2-oxo-2,3-dihydro-1H-Indol-3-yl]-N-éthyl-N-pyridin-3-ylméthyl-benzamide sous forme racémique, préparé à l'étape 1,1 et d'acétobromo-α-D-glucose,

SM [(+)ESI, m/z] = 766 (M⁺).
RMN ¹H 400MHz (CDCl₃) : 1,21 (m, 3H) ; 1,79 (s, 3H) ; 3,45 (s, 2H) ; 3,75-4,1 (m, 12H) ; 4,70-5,15 (m, 4H) ; 5,97 (m,1H) ; 6,40-9,50 (m, 13H).

### Exemple 5 :

Chlorure de 3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-1-(D-glactopyranosyl)-pyridinium
(I): R₀ = 2-MeO ; R₁ = 4-MeO ; R₂ = 5-Cl ; R₃= Me ; R₄ = 4-Cl ;

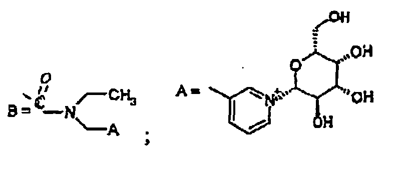

On obtient le produit attendu dans des conditions opératoires analogues à celles décrites à l'exemple 3 de la présente invention, à partir du 4-Chloro-3-[5-chloro-1-(2,4-diméthoxy-benzyl)-3-méthyl-2-oxo-2,3-dihydro-1H-indol-3-yl]-N-éthyl-N-pyridin-3-ylméthyl-benzamide sous forme racémique, préparé à l'étape 1.1 et d'acétobromo-α-D-galactose.
SM [(+)ESI, m/z] = 766 (M⁺).
RMN ¹H 400MHz (CDCl₃) : 1,18 (m, 3H) ; 1,77 (s, 3H) ; 3,42 (s, 2H) ; 3,76 (s, 3H) : 3,85 (s, 3H) ; 2,88-4,3 (m, 6H) ; 4,50-5,2 (m, 4H) ; 5,83 (m, 1 H) ; 6,35-9,40 (m, 13H).

### Exemple 6 :

4-Chloro-3-[5-chloro-1-(2,4-diméthoxy-benzyl)-3-méthyl-2-oxo-2,3-dihydro-1H-indol-3-yl]-N-éthyl-N-(1-oxy-pyrid-3-ylméthyl-benzamide
(I): R₀ = 2-MeO ; R₁ = 4-Me0 ; R₂ = 5-Cl ; R₃ = Me ; R₄ = 4-Cl ;

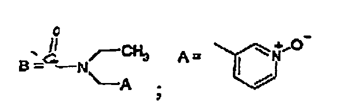

A 440 mg du composé de l'exemple 201 du document WO 01/074775 (en solution à -10°C dans 17 ml de 1,2-diméthoxyéthane), on ajoute 0.22 g d'acide 3-chloroperbenzoïque et on agite pendant 16 heures à environ 10°C. Le milieu est versé sur une solution aqueuse de bicarbonate de sodium et est extrait deux fois avec de l'acétate d'éthyle. Les phases organiques jointes sont séchées sur du sulfate de sodium puis évaporées à sec. On chromatographie sur une colonne de silice en éluant avec un gradient dlchlorométhane-méthanol 99/1 à 96/4. On obtient le produit attendu sous forme de base (résine blanche).

PF = 102°C
[α]D/2D = + 99,4° (c = 1, dans le MeOH).

### Exemple 7 :

Chlorhydrate du 4-chloro-3-[5-chloro-1-(2,4-diméthoxy-benzyl)-3-méthyl-2-oxo-2,3-dihydro-1H-indol-3-yl]-N-éthyl-N-(1-oxy-pyrid-3-ylméthyl)-benzamide
(I), Cl⁻ : R₀ = 2-MeO; R₁ = 4-MeO; R₂= 5-Cl ; R₃ = Me ; R₄ = 4-Cl ;

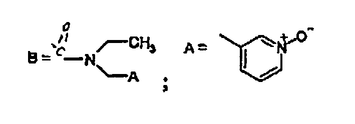

A 270 mg du produit préparé à l'exemple 6, on ajoute 1,8 ml d'une solution d'acide chlorhydrique (0,5 M) dans de l'éthanol. Après 1 heure d'agitation, on filtre, puis lave avec 3 ml d'éthanol, on sèche ensuite sous vide et on obtient le sel attendu sous forme de poudre blanche.

PF = 176°C.
SM [(+)ESI, m/z] = 620 (MH⁺).
RMN ¹H 250MHz (DMSO-d6) : 1,17 (t, 3H) ; 1,75 (s, 3H) ; 3,25-3,4 (m, 2H) ; 3,77 (s, 3H) ; 3,84 (s, 3H) ; 4,60-5,1 (m, 4H) ; 6,48-8,45 ( m, 13H).

### Exemple 8 :

(+)-4-Chloro-3-[5-chloro-1-(2,4-dimêthoxy-benzyl)-3-méthyl-2-oxo-2,3-dihydro-1H-indol-3-yl]-N-éthyl-N-[3-(1-oxy-pyridin-4-yl)-propyl]-benzamide
(I): R₀= 2-MeO ; R₁ = 4-MeO ; R₂= 5-Cl ; R₃ = Me ; R₄ = 4-Cl) ;

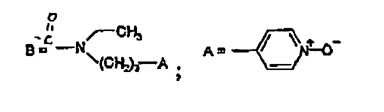

Par analogie avec le mode de préparation du composé de l'exemple 6 de la présente invention, et à partir du composé de l'exemple 204 du document WO 01/074775, on isole le produit attendu.

PF = 95°C
[α]D/20 + 97° (c = 1,MeOH).
SM [(+)ESI, m/z] = 648 (MH⁺).
RMN ¹H 250MHz (DMS4-d6): 1,15 (m, 3H) ; 1,74 (s, 3H) ; 1,93 (m, 2H) ; 2,40-2,80 (m, 2H) ; 3,15-3,60 (m, 4H) ; 3,77 (s, 3H) ; 3,84 (s, 3H) ; ; 4,65-5,01 (m, 2H) ; 6,40-8.20 (m, 13H).

Les composés selon l'invention ont fait l'objet d'études biochimiques et pharmacologiques. L'affinité des composés selon l'invention pour les récepteurs de l'ocytocine a été déterminée dans un test de liaison *in vitro* en utilisant la méthode décrite par J. Elands et al., Eur. J. Pharmacol., (1987), 147: pp. 197-207. Cette méthode consiste à étudier *in vitro* le déplacement d'un analogue radioiodé de l'ocytocine aux récepteurs de l'ocytocine dans une préparation membranaire de récepteurs ocytociques humains utérins. Les Cl₅₀ (concentration inhibitrice de 50 % de la liaison de l'analogue radioiodé de l'ocytocine à ses récepteurs) sont faibles et varient de 10⁻¹⁰ à 10⁻⁶ M dans ce dernier test. A titre d'exemple, le composé de l'exemple 2 présente une Cl₅₀ de 6,9 nM.

Les composés selon l'invention sont sélectifs des récepteurs de l'ocytocine.

L'affinité de ces composés pour les récepteurs humains V₁ₐ (méthode décrite par M. Thibonnier et al. dans J. Biol. Chem., (1994), 269, pp. 3304-3310), V_{1b} (méthode décrite par T. Sugimoto et al. dans J. Biol. Chem., (1994), 269, pp. 27088-27092) et V₂ (méthode décrite par T. Birnbaumer et al., dans Nature (Lond.), (1992), 357, pp.333-335) de la vasopressine a également été étudiée. Les composés étudiés sont peu ou pas affins pour les récepteurs V₁ₐ, V_{1b} et V₂. A titre indicatif, le composé de l'exemple 7 présente une Cl₅₀ inférieure à 50 nM avec un pourcentage d'inhibition inférieur à 50% lorsqu'il est testé à une concentration de 1 µM sur les récepteurs V₁ₐ, V_{1b} et V₂.

Le caractère agoniste ou antagoniste des composés est déterminé *in vitro* dans un test de mesure de calcium intracellulaire sur cellules exprimant les récepteurs ocytociques humains selon la technique générale décrite dans Am. J. Physiol., 268 et Heart Circ. Physiol., (1995), 37, H404-H410.

Lorsque les composés selon l'invention se comportent comme des antagonistes, leur Cl₅₀ est avantageusement comprise entre 0,5 µM et 0,5 nM. A titre d'exemple, le composé de l'exemple 3 est antagoniste avec une Cl₅₀ de 3 nM.

Les composés selon l'invention, ligands puissants et sélectifs des récepteurs de l'ocytocine sont particulièrement intéressants et ce, dans la prévention et/ou le traitement des désordres ocytocine-dépendants. Les composés selon la présente invention peuvent inhiber les effets de l'ocytocine.

Ils seront particulièrement intéressants dans la cicatrisation, dans l'analgésie et l'anxiolyse (prévention de la douleur et de l'anxiété), la dépression, la schizophrénie, l'autisme, le syndrome obsessionnel compulsif, l'agression, dans le comportement maternel (facilitation de la reconnaissance et de l'acceptation mère-enfant) et social, la mémoire, la régulation de la prise de nourriture et de boisson, la boulimie, la dépendance aux drogues, le sevrage et la motivation sexuelle. Ils pourront être utilisés avantageusement dans les désordres de la sphère urogénitale, notamment dans les domaines obstétrique et gynécologique, notamment comme agent tocolytique ou relaxant utérin ou pour contrôler les contractions de l'utérus avant que la grossesse soit arrivée à terme, lors du travail précoce ou prématuré chez la femme enceint, pour contrôler le travail prénatal, pour contrôler l'hyperactivité utérine au moment de la parturition en cas de détresse foetale (Lurie et al., Journal of Perinal Medicine, (2004), 32(2): pp. 137-139) ou encore contrôler le travail préparatoire en vue d'un accouchement par césarienne, pour résoudre les problèmes de stérilité, fertilité, contrôler les naissances (usage vétérinaire en particulier), contrôler l'oestrus, l'arrêt de l'allaitement, le sevrage, le transfert et l'implantation d'embryons ; traiter l'endométriose, les dysménorrhées ainsi que l'incontinence urinaire à l'effort ou d'urgence, l'hypertrophie bénigne de la prostate et les dysfonctions érectiles tel que l'éjaculation précoce, l'hypertension, l'hyponatrémie, l'insuffisance cardiaque, l'arthérosclérose, l'angiogénèse et réguler le stockage des graisses par l'adipocyte.

Par ailleurs, étant donné le rôle de l'ocytocine sur le contrôle de l'hormone lutéïnisante (J.J. Evans, J. Endocrin., (1996), 151 : pp. 169-174) les composés de l'invention pourront être utilisés pour induire la contraception.

De plus, les composés selon l'invention pourront être utilisés pour leurs effets antitumoraux, dans les tumeurs ocytocine sécrétantes, en particulier les cancers mammaires, pulmonaires et prostatiques. Ainsi, les composés selon l'invention pourront également être utilisés comme marqueurs de tumeur surexprimant les récepteurs de l'ocytocine (P. Cassoni, Annals of Oncology, (2001), 12(2): S37-S39 ; and B. Chini, British J. Of Cancer, (2003), 89 : pp. 930-936.

L'utilisation des composés selon l'invention pour la prévention et/ou le traitement des maladies ci-dessus mentionnées dans lesquelles l'ocytocine est impliquée, ainsi que pour la préparation de médicaments destinés à traiter ces maladies fait partie intégrante de l'invention.

La présente invention a donc également pour objet des compositions pharmaceutiques contenant au moins un composé selon l'invention et des excipients convenables. Lesdits excipients sont choisis selon la forme pharmaceutique et le mode d'administration souhaité : orale, sublinguale, sous-cutanée, intramusculaire, intra-veineuse, topique, intratrachéale, intranasale, transclermique, rectale ou intraoculaire. Notamment, le mode d'administration intra-veineuse pourra être choisi dans le cas de compositions pharmaceutiques destinées au traitement de désordres obstétriques et gynécologiques tel que l'hyperactivité utérine au moment de la parturition en cas de détresse foetale.

Les compositions pharmaceutiques sont préparées selon les techniques connues de l'homme de l'art.

Afin d'obtenir l'effet prophylactique ou thérapeutique désiré, chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Les composés selon l'invention pourront également être utilisés pour la préparation de compositions à usage vétérinaire destinées à réguler les naissances.

Les composés selon l'invention pourront aussi être utilisés pour la préparation de compositions cosmétiques. Ces formulations pourront se présenter sous forme de crème pour utilisation topique et seront destinées à contrôler la lipolyse, le stockage des graisses et la cellulite.

Lorsqu'on prépare une composition solide sous forme de comprimés, on mélange l'ingrédient actif principal avec un excipient pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières. Les comprimés peuvent être réalisés par différentes techniques, compression directe, granulation sèche, granulation humide ou fusion à chaud.

La présente invention selon un autre de ses aspects, concerne également une méthode de traitement des pathologies ci-dessus indiquées qui comprend l'administration d'un composé selon l'invention ou un de ses sels pharmaceutiquement acceptables.

## Revendications

1. Composé de formule (l): 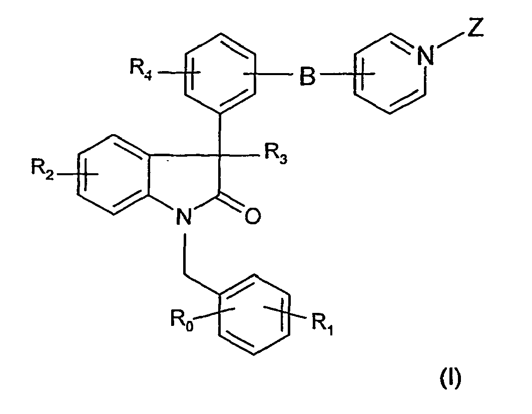 dans laquelle :
- R₀ représente un atome d'hydrogène, un atome d'halogène, un groupe (C₁-C₆)alkyle, un groupe (C₁-C₆)alcoxy ;
- R₁ représente un atome d'hydrogène, un atome d'halogène, un groupe (C₁-C₆)alkyle, un groupe (C₁-C₆)alcoxy ;
- R₂ représente un atome d'hydrogène, un atome d'halogène, un groupe (C₁-C₆)alkyle, un groupe (C₁-C₆)alcoxy ;
- R₃ représente un atome d'hydrogène, un groupe (C₁-C₆)alkyle ;
- R₄ représente un atome d'hydrogène, un atome d'halogène, un groupe (C₁-C₆)alkyle, un groupe (C₁-C₆)alcoxy ;
- Z représente :
- un atome d'oxygène,
- un groupe (C₁-C₆)alkyle éventuellement substitué par un ou plusieurs groupes hydroxyle, (C₁-C₆)alcoxy , -COOR dans lequel R représente un atome d'hydrogène ou un groupe (C₁-C₃)alkyle, -CONRR' dans lequel R et R' représentent indépendamment l'un de l'autre un atome d'hydrogène ou un groupe (C₁-C₃)alkyle ou bien R et R' forment ensemble avec l'atome d'azote auquel ils sont liés un groupe pyrrolidinyle, pipéridinyle ou morpholinyle,
- un groupe tétrahydrofuranyle ou un groupe tétrahydropyranyle, lesdits groupes étant éventuellement substitués par un ou plusieurs groupes (C₁-C₃)alkyle, hydroxyle, carboxyle ou un groupe -CH₂OH
- B représente un groupe T-W, dans lequel :
T représente un groupe -(CH₂)ₘ-, m pouvant être égal à 0 ou 1 ;
W représente :
- un groupe -CONR₆R₇ dans lequel :
• R₆ représente un atome d'hydrogène, un groupe (C₁-C₆)alkyle,
• R₇ représente un groupe (C₁-C₆)alkylène ;
- un groupe -NR₈COR₉ dans lequel :
• R₈ représente un atome d'hydrogène, un groupe (C₁-C₆)alkyle,
• R₉ représente un groupe -(CH₂)ₙ-, n pouvant prendre des valeurs de 0 à 3 ; à l'état de base ou de sel pharmaceutiquement acceptable, à l'état d'hydrate ou de solvate, ainsi que ses énantiomères, diastéréoisomères et leurs mélanges.

2. Composés de formule (I) selon la revendication 1, dans laquelle :
- R₀ représente un groupe (C₁-C₆)alcoxy ;
- R₁ représente un groupe (C₁-C₆)alcoxy ;
- R₂, R₃, R₄, Z et B sont tels que définis dans la revendication 1 ;
à l'état de base ou de sel pharmaceutiquement acceptable, à l'état d'hydrate ou de solvate, ainsi que ses énantiomères, diastéréoisomères et leurs mélanges.

3. Composés de formule (1) selon la revendication 1, dans laquelle :
- R₀ représente un atome d'hydrogène, un atome d'halogène, un groupe (C₁-C₆)alkyle, un groupe (C₁-C₆)alcoxy ;
- R₁ représente un atome d'hydrogène, un atome d'halogène, in groupe (C₁-C₆)alkyle, un groupe (C₁-C₆)alcoxy ;
- R₂, R₃, R₄ et B sont tels que définis dans la revendication 1,
- Z représente un atome d'oxygène ;
à l'état de base ou de sel pharmaceutiquement acceptable, à l'état d'hydrate ou de solvate, ainsi que ses énantiomères, diastéréoisomères et leurs mélanges.

4. Composés de formule (I) selon la revendication 1, dans laquelle :
- R₀ représente un atome d'hydrogène, un atome d'halogène, un groupe (C₁-C₆)alkyle, un groupe (C₁-C₆)alcoxy ;
- R₁ représente un atome d'hydrogène, un atome d'halogène, un groupe (C₁-C₆)alkyle, un groupe (C₁-C₆)alcoxy ;
- R₂, R₃, R₄ et B sont tels que définis dans la revendication 1 ;
- Z représente :
- un groupe (C₁-C₆)alkyle éventuellement substitué par un ou plusieurs groupes hydroxyle, (C₁-C₆)alcoxy , -COOR dans lequel R représente un atome d'hydrogène ou un groupe (C₁-C₃)alkyle, -CONRR' dans lequel R et R' représentent indépendamment l'un de l'autre un atome d'hydrogène ou un groupe (C₁-C₃)alkyle ou bien R et R' forment ensemble avec l'atome d'azote auquel ils sont liés un groupe pyrrolidinyle, pipéridinyle ou morpholinyle ;
- un groupe tétrahydrofuranyle ou un groupe tétrahydropyranyle, lesdits groupes étant éventuellement substitués par un ou plusieurs groupes (C₁-C₃)alkyle, un groupe hydroxyle, carboxyle ou un groupe -CH₂OH
à l'état de base ou de sel pharmaceutiquement acceptable, à l'état d'hydrate ou de solvate, ainsi que ses énantiomères, diastéréoisomères et leurs mélanges.

5. Composés selon la revendication 1, choisis parmi :
• Chlorure de 3-[({4-chloro-3-[5-chloro-1-(2,4-diméthoxy-benzyl)-3-méthyl-2-oxo-2,3-dihydro-1*H-*indol-3-yl]-benzoyl}-éthyl-amino)-méthyl]-1-(2-hydroxy-éthyl)-pyridinium,
• Chlorure de 1-carboxyméthyl-3-[({4-chloro-3-[5-chloro-1-(2,4-diméthoxy-benzyl)-3-méthyl-2-oxo-2,3-dihydro-1*H-*indol-3-yl]-benzoyl}-éthyl-amino)-éthyl]-pyridinium,
• Chlorure de 1-(β-D-glucopyranuronosyl))-3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-pyridinium,
• Chlorure de 3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-1-(D-glucopyranosyl)-pyridinium,
• Chlorure de 3-[({4-chloro-3-[5-chloro-1-(2,4-diméthoxy-benzyl)-3-méthyl-2-oxo-2,3-dihydro-1*H*-indol-3-yl]-benzoyl}-éthyl-amino)-méthyl]-1-(D-galactopyranosyl)-pyridinium,
• 4-Chloro-3-[5-chloro-1-(2,4-diméthoxy-benzyl)-3-méthyl-2-oxo-2,3-dihydro-1H-indol-3-yl]-N-éthyl-N-(1-oxy-pyrid-3-ylméthyl)-benzamide,
• Chlorhydrate du 4-chloro-3-[5-chloro-1-(2,4-diméthoxy-benzyl)-3-méthyl-2-oxo-2,3-dihydro-1H-indol-3-yl]-N-éthyl-N-(1-oxy-pyrid-3-ylméthyl)-benzamide,
• (+)-4-Chloro-3-[5-chloro-1-(2,4-diméthoxy-benzyl)-3-méthyl-2-oxo-2,3-dihydro-1H-indol-3-yl]-N-éthyl-N-[3-(1-oxy-pyridin-4-yl)-propyl]-benzamide.

6. Procédé de préparation d'un composé de formule (I) selon l'une quelconque des revendications 1 à 5, **caractérisé en ce que** l'on fait réagir le composé de formule (II) suivante : 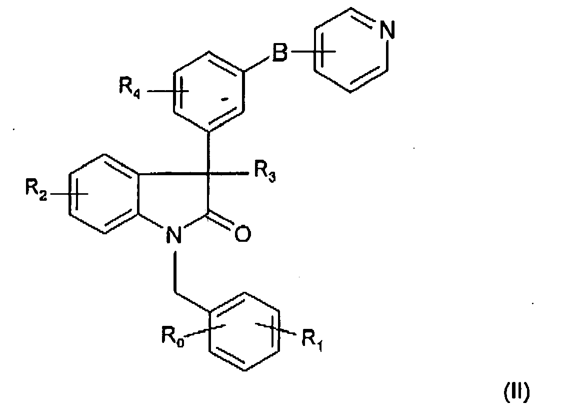 dans laquelle R₀, R₁, R₂, R₃, R₄ et B sont tels que définis précédemment, avec un peracide ou un composé de formule Z-X, dans lequel Z est tel que défini dans la revendication 1 et X représente un groupe partant.

7. Médicament **caractérisé en ce qu'**il consiste en au moins un composé de formulé (I) selon l'une quelconque des revendications 1 à 5.

8. Composition pharmaceutique, **caractérisée en ce qu'**elle comprend au moins un composé de formule (I) selon l'une quelconque des revendications 1 à 5.

9. Utilisation d'un composé de formule (I) selon l'une quelconque des revendications 1 à 5 pour la préparation d'un médicament tocolytique ou relaxant utérin.

10. Utilisation d'un composé de formule (I) selon l'une quelconque des revendications 1 à 5 pour la préparation de médicaments destinés au traitement et à la prévention des désordres ocytocine-dépendants, à savoir de médicaments destinés à favoriser la cicatrisation, traiter l'analgésie, l'anxiolyse, la dépression, la schizophrénie, l'autisme, le syndrome obsessionnel compulsif, l'agression, améliorer le comportement maternel et social, faciliter la reconnaissance et l'acceptation de la mère par l'enfant, traiter les troubles de la mémoire, réguler la prise de nourriture et de boisson, la dépendance aux drogues, le sevrage et la motivation sexuelle, traiter les désordres de la sphère urogénitale dans les domaines obstétrique et gynécologique, traiter le travail prématuré ou précoce chez la femme enceinte, contrôler les contractions de l'utérus avant que la grossesse soit arrivée à terme, contrôler le travail prénatal, contrôler l'hyperactivité utérine au moment de la parturition en cas de détresse foetale, traiter les dysménorrhées, contrôler le travail préparatoire en vue d'un accouchement par césarienne, résoudre les problèmes de stérilité, fertilité, contrôler les naissances, induire la contraception, contrôler l'oestrus, l'arrêt de l'allaitement, le sevrage, le transfert et l'implantation d'embryons, traiter l'endométriose, l'incontinence urinaire à l'effort ou d'urgence, l'hypertrophie bénigne de la prostate et les dysfonctions érectiles, l'éjaculation précoce, l'hypertension, l'hyponatrémie, l'insuffisance cardiaque, l'arthérosclérose, l'angiogénèse, réguler le stockage des graisses par l'adipocyte et la cellulite et traiter les cancers mammaires, pulmonaire ou prostatiques.

11. Utilisation d'un composé de formule (I) selon l'une quelconque des revendications 1 à 5 comme marqueurs de tumeur surexprimant les récepteurs de l'ocytocine.

## Claims

1. Compound of formula (I): 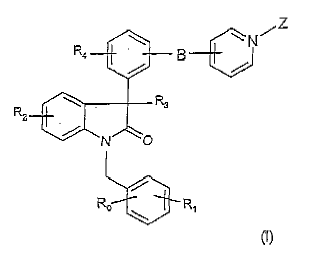 in which:
- R₀ represents a hydrogen atom, a halogen atom, a (C₁-C₆)alkyl group or a (C₁-C₆)alkoxy group;
- R₁ represents a hydrogen atom, a halogen atom, a (C₁-C₆)alkyl group or a (C₁-C₆)alkoxy group;
- R₂ represents a hydrogen atom, a halogen atom, a (C₁-C₆)alkyl group or a (C₁-C₆)alkoxy group;
- R₃ represents a hydrogen atom or a (C₁-C₆)alkyl group;
- R₄ represents a hydrogen atom, a halogen atom, a (C₁-C₆)alkyl group or a (C₁-C₆)alkoxy group;
- Z represents:
- an oxygen atom,
- a (C₁-C₆)alkyl group optionally substituted by one or more groups chosen from: hydroxyl, (C₁-C₆)alkoxy, -COOR, in which R represents a hydrogen atom or a (C₁-C₃)alkyl group, or -CONRR', in which R and R' represent, independently of one another, a hydrogen atom or a (C₁-C₃)alkyl group or else R and R' form, together with the nitrogen atom to which they are bonded, a pyrrolidinyl, piperidinyl or morpholinyl group;
- a tetrahydrofuranyl group or a tetrahydropyranyl group, said groups optionally being substituted by one or more (C₁-C₃)alkyl, hydroxyl or carboxyl groups or a -CH₂OH group;
- B represents a T-W group, in which:
T represents a (CH₂)ₘ- group, it being possible for m to be equal to 0 or 1;
Represents:
- a -CONR₆R₇ group, in which:
• R₆ represents a hydrogen atom or a (C₁-C₆)alkyl group,
• R₇ represents a (C₁-C₆)alkylene group;
- an -NR₈COR₉ group, in which:
R₈ represents a hydrogen atom or a (C₁-C₆)alkyl group,
• R₉ represents a -(CH₂)ₙ- group, it being possible for n to take values from 0 to 3;
in the form of the base or in the form of a pharmaceutically acceptable salt, in the hydrate or solvate form, and its enantiomers, diastereoisomers and their mixtures.

2. Compound of formula (I) according to Claim 1, in which:
- R₀ represents a (C₁-C₆)alkoxy group;
- R₁ represents a (C₁-C₆)alkoxy group;
- R₂, R₃, R₄, Z and B are as defined in Claim 1;
in the form of the base or in the form of a pharmaceutically acceptable salt, in the hydrate or solvate form, and its enantiomers, diastereoisomers and their mixtures.

3. Compound of formula (I) according to Claim 1, in which:
- R₀ represents a hydrogen atom, a halogen atom, a (C₁-C₆)alkyl group or a (C₁-C₆)alkoxy group;
- R₁ represents a hydrogen atom, a halogen atom, a (C₁-C₆) alkyl group or a (C₁-C₆) alkoxy group;
- R₂, R₃, R₄ and B are as defined in Claim 1;
- Z represents an oxygen atom;
in the form of the base or in the form of a pharmaceutically acceptable salt, in the hydrate or solvate form, and its enantiomers, diastereoisomers and their mixtures.

4. Compound of formula (I) according to Claim 1, in which:
- R₀ represents a hydrogen atom, a halogen atom, a (C₁-C₆)alkyl group or a (C₁-C₆)alkoxy group;
- R₁ represents a hydrogen atom, a halogen atom, a (C₁-C₆)alkyl group or a (C₁-C₆)alkoxy group;
- R₂, R₃, R₄ and B are as defined in Claim 1;
- Z represents:
- a (C₁-C₆)alkyl group optionally substituted by one or more groups chosen from: hydroxyl, (C₁-C₆)alkoxy, -COOR, in which R represents a hydrogen atom or a (C₁-C₃)alkyl group, or -CONRR', in which R and R' represent, independently of one another, a hydrogen atom or a (C₁-C₃)alkyl group or else R and R' form, together with the nitrogen atom to which they are bonded, a pyrrolidinyl, piperidinyl or morpholinyl group;
- a tetrahydrofuranyl group or a tetrahydropyranyl group, said groups optionally being substituted by one or more (C₁-C₃)alkyl groups, a hydroxyl or carboxyl group or a -CH₂OH group;
in the form of the base or in the form of a pharmaceutically acceptable salt, in the hydrate or solvate form, and its enantiomers, diastereoisomers and their mixtures.

5. Compound according to Claim 1, chosen from:
• 3-[({4-chloro-3-[5-chloro-1-(2,4-dimethoxybenzyl)-3-methyl-2-oxo-2,3-dihydro-1*H*-indol-3-yl]benzoyl}-(ethyl)amino)methyl]-1-(2-hydroxyethyl)pyridinium chloride
• 1-carboxymethyl-3-[({4-chloro-3-[5-chloro-1-(2,4-dimethoxybenzyl)-3-methyl-2-oxo-2,3-dihydro-1*H*-indol-3-yl] benzoyl} (ethyl)amino) methyl] pyridinium chloride,
• 1-(β-D-glucopyranuronosyl)-3-[({4-chloro-3-[5-chloro-1-(2,4-dimethoxybenzyl)-3-methyl-2-oxo-2,3-dihydro-1*H*-indol-3-yl]benzoyl} (ethyl)amino)methyl] pyridinium chloride,
• 3-[({4-chloro-3-[5-chloro-1-(2,4-dimethoxybenzyl)-3-methyl-2-oxo-2,3-dihydro-1*H*-indol-3-yl]benzoyl}-(ethyl)amino)methyl]-1-(D-glucopyranosyl)pyridinium chloride,
• 3-[({4-chloro-3-[5-chloro-1-(2,4-dimethoxybenzyl)-3-methyl-2-oxo-2,3-dihydro-1*H*-indol-3-yl]benzoyl}-(ethyl)amino)methyl]-1-(D-galactopyranosyl)pyridinium chloride,
• 4-chloro-3-[5-chloro-1-(2,4-dimethoxybenzyl)-3-methyl-2-oxo-2,3-dihydro-1*H*-indol-3-yl]-N-ethyl-N-(1-oxypyrid-3-ylmethyl)benzamide,
• 4-chloro-3-[5-chloro-1-(2,4-dimethoxybenzyl)-3-methyl-2-oxo-2,3-dihydro-1*H*-indol-3-yl]-N-ethyl-N-(1-oxypyrid-3-ylmethyl)benzamide hydrochloride,
• (+)-4-chloro-3-[5-chloro-1-(2,4-dimethoxybenzyl)-3-methyl-2-oxo-2,3-dxhydro-1*H*-indol-3-yl]-N-ethyl-N-[3-(1-oxypyridin-4-yl)propyl]benzamide.

6. Process for the preparation of a compound of formula (I) according to any one of Claims 1 to 5, **characterized in that** the compound of following formula (II) ; 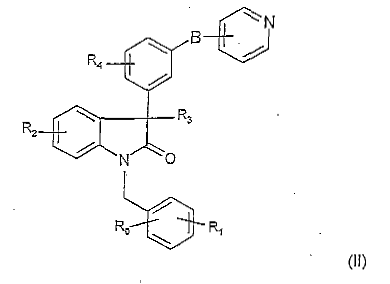 in which R₀, R₁, R₂, R₃, R₄ and B are as defined above, is reacted with a peracid or a compound of formula Z-X, in which Z is as defined in Claim 1 and X represents a leaving group.

7. Medicament, **characterized in that** it comprises at least one compound of formula (I) according to any one of Claims 1 to 5.

8. Pharmaceutical composition, **characterized in that** it comprises at least one compound of formula (I) according to any one of Claims 1 to 5.

9. Use of a compound of formula (I) according to any one of Claims 1 to 5 in the preparation of a uterine relaxant or tocolytic medicament.

10. Use of a compound of formula (I) according to any one of Claims 1 to 5 in the preparation of medicaments intended for the treatment and for the prevention of oxytocin-dependent disorders, namely medicaments intended to promote healing, to treat analgesia, anxiolysis, depression, schizophrenia, autism, obsessive compulsive syndrome or aggression, to improve maternal and social behaviour, to facilitate recognition and acceptance of the mother by the child, to treat memory disorders, to regulate food and drink intake, dependence on drugs, weaning and sexual motivation, to treat disorders of the urogenital sphere in the obstetric and gynaecological fields, to treat premature or early labour in pregnant women, to control contractions of the uterus before pregnancy has arrived at term, to control prenatal labour, to control uterine hyperactivity at the time of parturition in the event of foetal distress, to treat dysmenorrhoea, to control preparatory labour for the purpose of a caesarian delivery, to solve problems of sterility or fertility, to control births, to induce contraception, to control oestrus, the halting of breast feeding, weaning, or the transfer and implantation of embryos, to treat endometriosis, urinary stress or urgency incontinence, benign prostate hypertrophy, erectile dysfunctions, premature ejaculation, hypertension, hyponatraemia, cardiac insufficiency, atherosclerosis or angiogenesis, to regulate the storage of fat by the adipocyte and cellulitus and to treat breast, pulmonary or prostate cancers.

11. Use of a compound of formula (I) according to any one of Claims 1 to 5 as labels for tumours overexpressing oxytocin receptors.

## Patentansprüche

1. Verbindung der Formel (I): 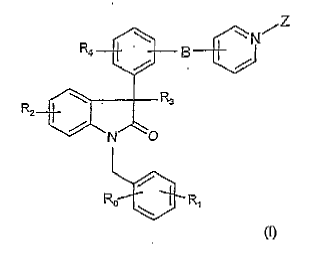 worin:
- R₀ für ein Wasserstoffatom, ein Halogenatom, eine (C₁-C₆)-Alkylgruppe oder eine (C₁-C₆)-Alkoxygruppe steht;
- R₁ für ein Wasserstoffatom, ein Halogenatom, eine (C₁-C₆)-Alkylgruppe oder eine (C₁-C₆)-Alkoxygruppe steht;
- R₂ für ein Wasserstoffatom, ein Halogenatom, eine (C₁-C₆)-Alkylgruppe oder eine (C₁-C₆)-Alkoxygruppe steht;
- R₃ für ein Wasserstoffatom oder eine (C₁-C₆)-Alkylgruppe steht;
- R₄ für ein Wasserstoffatom, ein Halogenatom, eine (C₁-C₆)-Alkylgruppe oder eine (C₁-C₆)-Alkoxygruppe steht;
- Z für:
- ein Sauerstoffatom,
- eine (C₁-C₆)-Alkylgruppe, die gegebenenfalls durch eine oder mehrere Hydroxylgruppen, (C₁-C₆)-Alkoxygruppen, -COOR-Gruppen, worin R für ein Wasserstoffatom oder eine (C₁-C₃)-Alkylgruppe steht, oder -CONRR'-Gruppen, worin R und R' unabhängig voneinander für ein Wasserstoffatom oder eine (C₁-C₃)-Alkylgruppe stehen oder R und R' gemeinsam mit dem stickstoffatom, an das sie gebunden sind, eine Pyrrolidinyl-, Piperidinyl-oder Morpholinylgruppe bilden, substituiert ist,
- eine Tetrahydrofuranylgruppe oder eine Tetrahydropyranylgruppe, wobei die Gruppen gegebenenfalls durch eine oder mehrere (C₂-C₃)-Alkylgruppen, Hydroxylgruppen, Carboxygruppen oder eine -CH₂OH-Gruppe substituiert sind,
steht;
- B für eine Gruppe T-W steht, worin:
T für eine -(CH₂)ₘ-Gruppe steht, wobei m gleich 0
oder 1 sein kann;
W für:
- eine -CONR₆R₇-Gruppe, worin;
• R₆ für ein Wasserstoffatom oder eine (C₁-C₆)-Alkylgruppe steht,
• R₇ für eine (C₁-C₆)-Alkylengruppe steht,
- eine -NR₈COR₉-Gruppe, worin:
• R₈ für ein Wasserstoffatom oder eine (C₁-C₆)-Alkylgruppe steht,
• R₉ für eine -(CH₂)ₙ-Gruppe steht, wobei n Werte von 0 bis 3 annehmen kann;
steht;
in Basenform oder in Form eines pharmazeutisch annehmbaren Salzes, in Hydrat- oder Solvatform sowie Enantiomere, Diastereomere und Gemische davon.

2. Verbindung der Formel (I) nach Anspruch 1, worin:
- R₀ für eine (C₁-C₆)-Alkoxygruppe steht;
- R₁ für eine (C₁-C₆)-Alkoxygruppe steht;
- R₂, R₃, R₄, Z und B die in Anspruch 1 angegebene Bedeutung besitzen;
in Basenform oder in Form eines pharmazeutisch annehmbaren Salzes, in Hydrat- oder Solvatform sowie Enantiomere, Diastereomere und Gemische davon.

3. Verbindung der Formel (I) nach Anspruch 1, worin:
- R₀ für ein Wasserstoffatom, ein Halogenatom, eine (C₁-C₆)-Alkylgruppe oder eine (C₁-C₆)-Alkoxygruppe steht;
- R₁ für ein Wasserstoffatom, ein Halogenatom, eine (C₁-C₆)-Alkylgruppe oder eine (C₁-C₆)-Alkoxygruppe steht;
- R₂, R₃, R₄ und B die in Anspruch 1 angegebene Bedeutung besitzen;
- Z für ein Sauerstoffatom steht;
in Basenform oder in Form eines pharmazeutisch annehmbaren Salzes, in Hydrat- oder Solvatform sowie Enantiomere, Diastereomere und Gemische davon.

4. Verbindung der Formel (I) nach Anspruch 1, worin:
- R₀ für ein Wasserstoffatom, ein Halogenatom, eine (C₁-C₆)-Alkylgruppe oder eine (C₁-C₆)-Alkoxygruppe steht;
- R₁ für ein Wasserstoffatom, ein Halogenatom, eine (C₁-C₆)-Alkylgruppe oder eine (C₁-C₆)-Alkoxygruppe steht;
- R₂, R₃, R₄ und B die in Anspruch 1 angegebene Bedeutung besitzen;
- Z für:
- eine (C₁-C₆)-Alkylgruppe, die gegebenenfalls durch eine oder mehrere Hydroxylgruppen, (C₁-C₆)-Alkoxygruppen, -COOR-Gruppen, worin R für ein Wasserstoffatom oder eine (C₁-C₃)-Alkylgruppe steht, oder -CONRR'-Gruppen, worin R und R' unabhängig voneinander für ein Wasserstoffatom oder eine (C₁-C₃)-Alkylgruppe stehen oder R und R' gemeinsam mit dem Stickstoffatom, an das sie gebunden sind, eine Pyrrolidinyl-, Piperidinyl-oder Morpholinylgruppe bilden, substituiert ist,
- eine Tetrahydrofuranylgruppe oder eine Tetrahydropyranylgruppe, wobei die Gruppen gegebenenfalls durch eine oder mehrere (C₁-C₃)-Alkylgruppen, Hydroxylgruppen, Carboxylgruppen oder eine -CH₂OH-Gruppe substituiert sind,
steht;
in Basenform oder in Form eines pharmazeutisch annehmbaren Salzes, in Hydrat- oder Solvatform sowie Enantiomere, Diastereomere und Gemische davon.

5. Verbindung nach Anspruch 1, ausgewählt unter:
• 3-[({4-Chlor-3-[5-chlor-1-(2,4-dimethoxybenzyl)-3-methyl-2-oxo-2,3-dihydro-1*H*-indol-3-yl]benzoyl}(ethyl)amino)methyl]-1-(2-hydroxyethyl)pyridiniumchlorid,
• 1-Carboxymethyl-3-[({4-chlor-3-[5-chlor-1-(2,4-dimethoxybenzyl)-3-methyl-2-oxo-2,3-dihydro-1*H-*indol-3-yl]benzoyl}(ethyl)amino)methyl]-pyridiniumchlorid,
• 1-(β-D-Glucopyranuronosyl)-3-[({4-chlor-3-[5-chlor-1-(2,4-dimethoxybenzyl)-3-methyl-2-oxo-2,3-dihydro-1*H*-indol-3-yl]benzoyl}(ethyl)-amino)methyl]pyridiniumchlorid,
• 3-[({4-Chlor-3-[5-chlor-1-(2,4-dimethoxybenzyl)-3-methyl-2-oxo-2,3-dihydxo-1*H*-indol-3-yl]benzoyl}(ethyl)amino)methyl]-1-(D-gluco-pyranosyl)pyridiniumchlorid,
• 3-[({4-Chlor-3-[5-chlor-1-(2,4-dimethoxybenzyl)-3-methyl-2-oxo-2,3-dihydro-1*H*-indol-3-yl]benzoyl}(ethyl)amino)methyl]-1-(D-galacto-pyranosyl)pyridiniumchlorid,
• 4-Chlor-3-[5-chlor-1-(2,4-dimethoxybenzyl)-3-methyl-2-oxo-2,3-dihydro-1*H*-indol-3-yl]-N-ethyl-N-(1-oxypyrid-3-ylmethyl)benzamid,
• 4-Chlor-3-[5-chlor-1-(2,4-dimethoxybenzyl)-3-methyl-2-oxo-2,3-dihydro-1*H*-indol-3-yl]-N-ethyl-N-(1-oxypyrid-3-ylmethyl)benzamid-hydrochlorid,
• (+)-4-Chlor-3-[5-chlor-1-(2,4-dimthoxybenzyl) - 3-methyl-2-oxo-2,3-dihydro-1*H*-indol-3-yl]-N-ethyl-N-[3-(1-oxypyridin-4-yl)propyl]benzamid.

6. Verfahren zur Herstellung einer Verbindung der Formel (I) nach einem der Ansprüche 1 bis 5, **dadurch gekennzeichnet, daß** man die Verbindung der folgenden Formel (II): 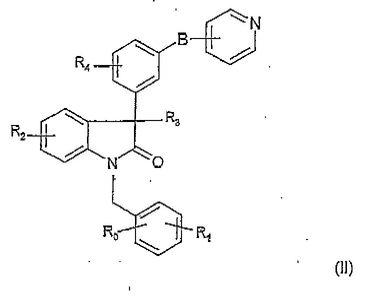 worin R₀, R₁, R₂, R₃, R₄ und B die oben angegebene Bedeutung besitzen, mit einer Persäure oder einer Verbindung der Formel Z-X, worin Z die in Anspruch 1 angegebene Bedeutung besitzt und X für eine Abgangsgruppe steht, umsetzt.

7. Medikament, **dadurch gekennzeichnet, daß** es aus mindestens einer Verbindung der Formel (I) nach einem der Ansprüche 1 bis 5 besteht.

8. Pharmazeutische Zusammensetzung, **dadurch gekennzeichnet, daß** sie mindestens eine Verbindung der Formel (I) nach einem der Ansprüche 1 bis 5 enthält.

9. Verwendung einer Verbindung der Formel (I) nach einem der Ansprüche 1 bis 5 zur Herstellung eines tocolytischen oder uterusrelaxierenden Medikaments.

10. Verwendung einer Verbindung der Formel (I) nach einem der Ansprüche 1 bis 5 zur Herstellung von Medikamenten zur Behandlung und Prävention von Oxytocin-abhängigen Störungen, d.h. Medikamenten zur Förderung der Vernarbung, zur Behandlung von Analgesie, Anxiolyse, Depression, Schizophrenie, Autismus, obsessiv-kompulsivem Syndrom oder Aggression, zur Verbesserung des mütterlichen und sozialen Verhaltens, zur Erleichterung des Wiedererkennens und der Annahme der Mutter durch das Kind, zur Behandlung von Gedächtnisstörungen, zur Regulierung der Aufnahme von Nahrung und Getränken, Drogenabhängigkeit, sexuellem Entzug und sexueller Motivation, zur Behandlung von Störungen im Urogenitalbereich auf den Gebieten der Obstetrik und Gynäkologie, zur Behandlung von Frühgeburt und Frühwehen bei der Schwangeren, zur Steuerung der Kontraktionen des Uterus vor dem errechneten Ende der Schwangerschaft, zur Steuerung der pränatalen Wehen, zur Steuerung der Uterushyperaktivität zum Zeitpunkt der Geburt im Fall von fetaler Notsituation, zur Behandlung von Dysmenorrhoen, zur Steuerung der vorbereitenden Wehen im Hinblick auf eine Kaiserschnittentbindung, zur Lösung von Sterilitäts- oder Fertilitätsproblemen, zur Geburtenkontrolle, zur Induzierung der Kontrazeption, zur Steuerung des Östrus, des Endes des Stillens, des Abstillens oder des Transfers und der Implantation von Embryos, zur Behandlung von Endometriose, Belastungs- oder Drangharninkontinenz, gutartiger Prostatahypertrophie und Erektionsdysfunktionen, vorzeitiger Ejakulation, Hypertonie, Hyponatriämie, Herzinsuffizienz, Atherosklerose oder Angiogenese, zur Regulierung der Fettspeicherung durch Adipozyten und Cellulite und zur Behandlung von Brust-, Lungen- oder Prostatakarzinomen.

11. Verwendung einer Verbindung der Formel (I) nach einem der Ansprüche 1 bis 5 als Marker für Tumore, die Oxytocinrezeptoren überexprimieren.
